# EUROPEAN PATENT APPLICATION

(11) **EP 2 659 909 A2**
(43) Date of publication of application: **06.11.2013**
(21) Application number: 13165960.9
(22) Date of filing: 04.02.2005
(51) Int. Cl.: A61K 41/00, A61N 5/06, A61P 17/00, A61P 17/10, A61K 31/409, A61K 31/555, A61K 31/203, A61K 31/07

(54) **Photodynamic therapy for the treatment of acne**

(30) Priority: 06.02.2004 CA 2457214; 09.06.2004 CA 2470403
(62) Divisional of application: 12170201.3
(71) Applicant: QLT Inc., Vancouver, British Columbia V5T 4T5 (CA)
(72) Inventor: The designation of the inventor has not yet been filed
(74) Representative: Lau, Sarah Jane

(57) **Abstract**

The present method involves the photodynamic treatment of acne vulgaris. The method involves the topical administration of a photosensitizer composition comprising hydrophobic green porphyrins such as lemuteporfin, polyethylene glycol and skin penetration enhancers such as oleyl alcohol and TRANSCUTOL™ to acne-affected skin and subsequent exposure of that skin to energy of a wavelength of activating the photosensitizer.

## Description

### FIELD OF THE INVENTION

This invention relates to a method of treating acne with photodynamic therapy (PDT). The use of PDT and appropriate photosensitizers for treating acne, especially acne vulgaris, is contemplated and disclosed.

### BACKGROUND OF THE INVENTION

Acne is a common dermatological condition affecting many people. Although often transitory in nature, acne can be associated with long-term consequences such as psychological and/or physical scarring. Clinical manifestations of acne includes comedones for mild lesions, papules, pustules, and nodules for more severe inflammatory lesions. The pathogenesis of acne is multi-factorial. It can involve an increase in keratinocytes, desquamation, hyperactive sebaceous glands with increased sebum production, *Propionibacterium acnes* proliferation and local inflammatory responses.

There are an array of therapies for acne targeting different and in some cases multiple pathogenic factors. Topical agents such as retinoids and benzoyl peroxide can be used for treating mild to moderate acne and are known to be able to remove comedones, kill bacteria and reduce inflammation. Antibiotics, given either topically or orally, can be used for treating mild to moderate acne. Light-based treatments such as 420-nm blue light or 1450-nm thermal lasers can also be used to treat mild to moderate acne. Accutane™ is an orally administrated retinoic acid that has been approved for treating severe, recalcitrant and nodular acne. It can be efficacious at removing comedones, reducing inflammation and inhibiting proliferation, differentiation and lipogenesis of sebaceous glands.

However, there are significant deficiencies associated with currently available therapies. Topical therapies are only marginally effective against mild to moderate acne and can be associated with local irritation. The use of antibiotics is associated with development of drug-resistant bacteria. Accutane is a known teratogenic agent and is associated with multiple significant systemic toxicities including increased risk of depression, increase in blood lipid and significant mucocutaneous adverse effects. Therefore, there is a need for novel therapeutic approaches with good efficacy and safety profiles.

Photodynamic therapy (PDT) has been proposed as a possible treatment for acne. For example, US Patent Number 5,095,030 (Levy) mentions acne as a possible indication which may be treated with PDT. Other disclosures which mention acne as a possible indication for treatment with PDT include WO03/86460 (Geronemus), WO03/39597 (Boch), WO02/13788 (Anderson), US2001/0023363 (Harth), US6645230 (Whitehurst), US6626932 (Whitehurst), and US5955490 (Kennedy). A more detailed discussion can be found in "Topical ALA-Photodynamic Therapy for the Treatment of Acne Vulgaris" J. Invest Dermatol 115:183-192, 2000. This paper discusses the use of the photosensitizer ALA to treat acne vulgaris. However, the paper discusses serious adverse events that occurred during and after the treatment including erythema, edema, and sensations of pain, burning and itching.

Citation of the above documents is not intended as an admission that any of the foregoing is pertinent prior art. All statements as to the date or representation as to the contents of these documents is based on the information available to the applicant and does not constitute any admission as to the correctness of the dates or contents of these documents.

### SUMMARY OF THE INVENTION

The present invention relates to a photodynamic method of treating acne vulgaris. The method comprises:
(i) delivering photosensitizer to tissue affected by acne vulgaris; and
(ii) exposing the tissue to energy of a wavelength capable of activating the photosensitizer.

While not wishing to be bound by theory, it is believed that PDT works to treat acne through at least two mechanisms. First, PDT has an antibacterial effect and, second, it reduces the size and/or activity of the sebaceous glands.

### DETAILED DESCRIPTION OF THE INVENTION

The present method involves the photodynamic treatment of acne vulgaris. The method involves the administration of photosensitizer to acne affected skin and subsequent exposure of that skin to energy of a wavelength capable of activating the photosensitizer. The method can also be used as a prophylactic treatment for skin that is suspected of being vulnerable to acne. Therefore, as used herein the term "exhibiting symptoms of acne" includes skin having lesions and skin that is thought to be vulnerable to developing lesions in the future. The method may be used to treat mild, moderate or severe acne and all types of acne lesions. It is preferred that the acne affected subject receiving treatment is at least 12 years of age.

In one aspect of the present invention the method involves:
(i) topically applying a composition comprising a green porphyrin and a solublizer to skin tissue exhibiting symptoms of acne, and
(ii) exposing the tissue to energy of a wavelength capable of activating the photosensitizer.

It has surprisingly been found that topical formulations comprising green porphyrins and solublizer can penetrate into the hair follicle and sebaceous gland but are only found at low levels in other, surrounding tissues. It has also been found that, when compared to other photosensitizers such as ALA, green porphyrins show a surprising lack of adverse events. While not wishing to be bound by theory, it is believed that this selectivity avoids unwanted skin reactions to the photodynamic therapy. Preferred photodynamic treatment methods, compositions, and parameters are described in more detail below.

In another aspect the method involves:
(i) topically applying a composition comprising a photosensitizer to skin tissue exhibiting symptoms of acne,
(ii) removing excess composition from the skin, and
(iii) exposing the tissue to energy of a wavelength capable of activating the photosensitizer.

It has surprisingly been found that removing the excess photosensitizer does not compromise the efficacy of the treatment and may help avoid unwanted side effects. Preferred photodynamic treatment methods, compositions, and parameters are described in more detail below. The excess composition can be removed by any suitable method. Preferred methods include wiping with dry cloth, wiping with a moist towelette, washing with alcohol, washing with a soap free cleanser, washing with a mild soap cleanser, and combinations thereof. A preferred method of removing the excess composition is to wash the skin with mild shampoo such as Cliniderm.

It is also believed that the removal of the excess composition could avoid the photosensitizer in the excess creating a 'shadow' which would prevent the activation energy from reaching the target tissue (e.g. the sebaceous gland).

In another aspect the method involves:
(i) topically applying a composition comprising a green porphyrin and solublizer to skin tissue exhibiting symptoms of acne, and
(ii) exposing the tissue to energy of a wavelength capable of activating the photosensitizer,
wherein the treatment is repeated until the total number of acne lesions has been reduced by 10% or more. Preferably, the total number of lesions is reduced by 15%, 20%, 25%, 30%, 35%, 40%, 45%, 50%, 60%, 70%, 80%, 90%, or more. The total number of lesions can be assessed by predefining one of more test area(s) before commencement of the treatment. Lesion counts (non-inflammatory, inflammatory, and total) are performed within the test area(s). Sizes of the of the lesions within the test area are also recorded. The test areas are also photographed. To be representative a number of test areas are selected per patient and these may vary depending on the anatomical distribution of the lesions of that patient. The test areas are reassessed one day, one week, two weeks and one month after completion of the photodynamic therapy. The reduction in lesion count is then calculated. Preferred photodynamic treatment methods, compositions, and parameters are described in more detail below

In another aspect the method involves:
(i) photodynamically treating skin exhibiting symptoms of acne, and
(ii) treating the same patient with topical retinoids, oral retinoids, antibiotics (especially topical), oral contraceptives, topical anti-androgens, blue light therapy, laser therapy, or combinations thereof.

While not wishing to be bound by theory, it is believed that PDT has a beneficial effect when combined with other therapies. Non-limiting examples of suitable therapies for being combined with PDT include tazarotene, isotretinoin, clindamycin, atrisone (from Atrix Labs), MBI594AN (from Micrologix), Smoothbean 1450 nm Laser therapy, and Blue-light PhotoTherapy.

A preferred embodiment of this aspect is to combine PDT treatment with retinoid treatment, especially topical retinoid treatment.

One embodiment of this aspect involves a composition comprising photosensitizer and at least one other topical agent used in the treatment of acne such as retinol. In this way the active agents may be delivered at the same time avoiding the necessity of applying two compositions to the same area.

Preferred photodynamic treatment methods, compositions, and parameters are described in more detail below.

In another aspect the method involves:
(i) topically applying a composition comprising a green porphyrin and solublizer to skin tissue exhibiting symptoms of acne, and
(ii) exposing the tissue to energy at a wavelength capable of activating the photosensitizer,
wherein the activation energy is at least in part supplied by light emitting diodes. The LED are preferably arrayed in a manner that somewhat follows the contours of the skin to be treated. A preferred arrangement is multiple flat panels of LED's that are moveable so that they can be positioned appropriately. As mentioned above, PDT can be combined with Blue-light Phototherapy to give extra efficacy benefits. Therefore, one embodiment of this aspect of the invention involves the activation energy being delivered by an LED device that supplies both red (e.g. 600-750nm) and blue light (e.g. 390-450nm). A preferred embodiment supplies light at about 420nm and at about 690nm. Preferred photodynamic treatment methods, compositions, and parameters are described in more detail below.

In another aspect the method involves:
(i) topically applying a composition comprising a green porphyrin to skin tissue exhibiting symptoms of acne,
(ii) exposing the tissue to energy at a wavelength capable of activating the photosensitizer,
(iii) photodynamically treating the tissue with another, non-green porphyrin photosensitizer.

Preferred photodynamic treatment methods, compositions, and parameters are described in more detail below.

### Photodynamic Therapy

Preferably, the photosensitizer herein is delivered topically to the target tissue. Topical delivery avoids some of the photosensitivity issues associated with systemic delivery of photosensitizers. When the photosensitizer is applied topically it may be applied to the acne lesions alone or, preferably, to the acne lesions and to surrounding unaffected tissues.

Any suitable photosensitizing agent or mixture of agents may be used herein. Typically, these agents will absorb radiation in the range of from 400nm to 900nm, preferably from 600nm to 750nm.

As used herein, "photosensitizer" or "photosensitizing agent" means a chemical compound that absorbs electromagnetic radiation, most commonly in the visible spectrum, and releases it as another for of energy, most commonly as reactive oxygen species and/or as thermal energy. Preferably, the compound is nontoxic to humans or is capable of being formulated in a nontoxic composition. Preferably, the chemical compound in its photodegraded form is also nontoxic. A non-exhaustive list of photosensitive chemicals may be found in Kreimer-Birnbaum, Sem. Hematol. 26:157-73, 1989 and in Redmond and Gamlin, Photochem. Photbiol. 70 (4): 391-475 (1999) both of which are incorporated herein by reference.

There are a variety of preferred synthetic and naturally occurring photosensitizers, including, but not limited to, pro-drugs such as the pro-porphyrin 5-aminolevulinic acid (ALA) and derivatives thereof, porphyrins and porphyrin derivatives e.g. chlorins, bacteriochlorins, isobacteriochlorins, phthalocyanine and naphthalocyanines and other tetra- and poly-macrocyclic compounds, and related compounds (e.g. pyropheophorbides, sapphyrins and texaphyrins) and metal complexes (such as, but not limited by, tin, aluminum, zinc, lutetium) Tetrahydrochlorins, purpurins, porphycenes, and phenothiaziniums are also within the scope of the invention. Other suitable photosensitizers include bacteriochlorophyll derivatives such as those described in WO-A-97/19081, WO-A-99/45382 and WO-A-01/40232. A preferred bacteriochlorophyll is palladium-bacteriopheophorbide WST09 (Tookad™). Preferably the photosensitizers are selected from pro-porphyrins, porphyrins, and mixtures thereof. Some examples of pro-drugs include aminolevulinic acid such as Levulan™ and aminolevulinic acid esters such as described in WO-A-02/10120 and available as Metvix™, Hexvix™ and Benzvix™. Some examples of di-hydro or tetra-hydro porphyrins are described in EP-A-337,601 or WO-A-01/66550 and available as Foscan™ (temoporfin). Combinations of two or more photosensitizers may be used in the practice of the invention.

In certain embodiments it is preferred that the photosensitizers are selected from those which photobleach upon exposure to activation energy.

A particularly potent group of photosensitizers is known as green porphyrins, which are described in detail in U.S. Patent No. 5,171,749 (incorporated herein by reference). The term "green porphyrins" refers to porphyrin derivatives obtained by reacting a porphyrin nucleus with an alkyne in a Diels-Alder type reaction to obtain a mono-hydrobenzoporphyrin. Such resultant macropyrrolic compounds are called benzoporphyrin derivatives (BPDs), which is a synthetic chlorin-like porphyrin with various structural analogues, as shown in U.S. Patent 5,171,749. Typically, green porphyrins are selected from a group of tetrapyrrolic porphyrin derivatives obtained by Diels-Alder reactions of acetylene derivatives with protoporphyrin under conditions that promote reaction at only one of the two available conjugated, nonaromatic diene structures present in the protoporphyrin-IX ring systems (rings A and B). Metallated forms of a Gp, in which a metal cation replaces one or two hydrogens in the center of the ring system, may also be used in the practice of the invention. The preparation of the green porphyrin compounds useful in this invention is described in detail in U.S. Patent No. 5,095,030 (incorporated herein by reference). Preferred green porphyrins include benzoporphyrin derivative diester diacid (BPD-DA), mono-acid ring A (BPD-MA), mono-acid ring B (BPD-MB), or mixtures thereof. These compounds absorb light at about 692nm wavelength which has good tissue penetration properties. The compounds of formulas BPD-MA and BPD-MB may be homogeneous, in which only the C ring carbalkoxyethyl or only the D ring carbalkoxyethyl would be hydrolyzed, or may be mixtures of the C and D ring substituent hydrolyzates. A number of other BPD B-ring derivatives may also be used in the present invention. These derivatives have the following general formula: 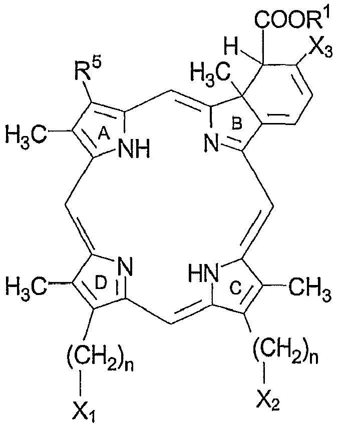 wherein; R⁵ is vinyl, R¹ and R⁶ are methyl, and n is 2. X₁, X₂, and X₃ are listed in the tables below:

**Table 1. Hydrophilic BPD B-ring analogs**

| Drug | X₁ | X₂ | X₃ |
|---|---|---|---|
| QLT0061 | COOH | COOH | COOH |
| QLT0077 | CONH(CH₂)₂N⁺(CH₃)₃I- | CONH(CH₂)₂N⁺(CH₃)₃I- | COOCH₃ |
| QLT0079 | CONH(CH₂)₂N⁺(CH₃)₂((CH₂)₃CH | CONH(CH₂)₂N⁺(CH₃)₂((CH₂)₃CH₃) | COOCH₃ |
| QLT0086 | CONHCH(COOH)CH₂COOH | CONHCH(COOH)CH₂COOH | COOCH₃ |
| QLT0092 | CONH(CH₂)₂NH(CH₃)₂ | CONH(CH₂)₂NH(CH₃)₂ | COOCH₃ |
| QLT0094 | CF₃COO-CONHCH₂COOH | CF₃COO-CONHCH₂COOH | CONHCH₂COOH |

**Table 2. Lipophilic BPD B-ring analogs**

| Drug | X1 | X2 | X3 |
|---|---|---|---|
| QLT0060 | CO(O(CH₂)₂)OH | CO(O(CH₂)₂)OH | COOCH₃ |
| QLT0069 | COOCH₃ | COOCH₃ | COOH |
| QLT0078 | CO(O(CH₂)₂)₂OH | CO(O(CH₂)₂)₂OH | COOCH₃ |
| QLT0080 | CO(O(CH₂)₂)₃OH | CO(O(CH₂)₂)₂OH | COOCH₃ |
| QLX0081 | CO(O(CH₂)₂)₂OCH₃ | CO(O(CH₂)₂)₂OCH₃ | CO(O(CH₂)₂)₂OCH₃ |
| QLT0082 | CO(O(CH₂)₂)₂OH | CO(O(CH2)₂)₂)₂OH | CO(O(CH₂)₂)₂OH |
| QLT0083 | CO(O(CH₂)₂)₃OH | CO(O(CH₂)₂)₃OH | CO(O(CH₂)₂)₃OH |
| QLT0087 | CO(O(CH₂)₂)₄OH | CO(O(CH₂)₂)₄OH | COOCH₃ |
| QLT0088 | COOCH₃ | COOCH₃ | CONH(C₆H₄)(C₅H₁₀N) |
| QLT0090 | CO(O(CH₂)₂)₅OH | CO(O(CH₂)₂)₂OH | COOCH₃ |
| QLT0093 | CO(O(CH₂)₂)₅OH | CO(O(CH₂)₂)₂OH | CO(O(CH₂)₂)₅OH |

Preferred photosensitizers include verteporfin the benzoporphyrin derivative mono-acid (BPD-MA), lemuteporfin (QLT0074 as set forth in U.S. Pat. No. 5,929,105 referred to therein as A-EA6) and B3 (as set forth in U.S. Pat. No. 5,990,149). A highly preferred photosensitizer is lemuteporfin which has the structure:

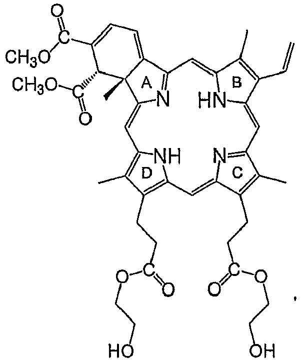

Additionally, the photosensitizers may be conjugated to various ligands to facilitate targeting. These ligands include receptor-specific peptides and/ore ligands as well as immunoglobulins and fragments thereof. Preferred ligands include antibodies in general and monoclonal antibodies, as well as immunologically reactive fragments of both.

Dimeric forms of the green porphyrin and dimeric or multimeric forms of green porphyrin/porphyrin combinations can be used. The dimers and oligomeric compounds of the invention can be prepared using reactions analogous to those for dimerization and oligomerization of porphyrins *per se.* The green porphyrins or green porphyrin/porphyrin linkages can be made directly, or porphyrins may be coupled, followed by a Diels-Alder reaction of either or both terminal porphyrins to convert them to the corresponding green porphyrins.

In addition to the above mentioned photosensitizing agents, other examples of photosensitizers include, but are not limited to, green porphyrins disclosed in US Pat. Nos. 5,283,255, 4,920,143, 4,883,790, 5,095,030, and 5,171,749; and green porphyrin derivatives, discussed in US Pat. Nos. 5,880,145 and 5,990,149. Several structures of typical green porphyrins are shown in the above cited patents, which also provide details for the production of the compounds.

The photosensitizer containing preparations of the invention may be administered systemically or locally and may be used alone or as components of mixtures. Preferably the administration is local. The route of administration for the photosensitizer may be topical, intradermal, intravenous, oral, or by use of an implant. Preferably the route of administration is topical. For example, the photosensitizer may be administered by means including, but not limited to, topical lotions, topical creams, topical pastes, topical suspensions, intradermal injection or via an implant. Preferred are topical lotions, topical creams, and topical pastes.

The photosensitizers may be formulated into a variety of compositions. These compositions may comprise any component that is suitable for the intended purpose, such as conventional delivery vehicles and excipients including isotonising agents, pH regulators, solvents, solubilizers, dyes, gelling agents and thickeners and buffers and combinations thereof. Pharmaceutical formulations suitable for use with the instant photosensitizers can be found, for instance, in Remington's Pharmaceutical Sciences. Preferred formulations herein comprise pharmaceutical excipients or carriers capable of directing the photosensitizer to the sebaceous gland. Suitable excipients for use with photosensitizers include water, saline, dextrose, glycerol and the like.

Typically, the photosensitizer is formulated by mixing it, at an appropriate temperature, e.g., at ambient temperatures, and at appropriate pHs, and the desired degree of purity, with one or more physiologically acceptable carriers, i.e., carriers that are nontoxic at the dosages and concentrations employed.

Preferred formulations are described in WO03/39597. The formulations preferably comprise a skin-penetration enhancer. Any skin-penetration enhancer suitable for aiding the delivery of the photosensitizing agent can be used herein. A list of skin-penetration enhancers can be found in "Pharmaceutical Skin Penetration Enhancement" (1993) Walters, K.A., ed.; Hadgraft, J., ed-New York, N.Y. Marcel Dekker and in "Skin Penetration Enhancers cited in the Technical Literature" Osbourne, D.W. Pharmaceutical Technology, November 1997, pp 59-65, both of which are incorporated herein by reference. Preferred for use in the formulations herein are hydrophobic skin-penetration enhancers. Preferred skin-penetration enhancers are selected from glycol ethers, fatty acids, fatty acid esters, glycol esters, glycerides, azones, polysorbates, alcohols, dimethylsulfoxide, and mixtures thereof. Preferred skin-penetration enhancers for use herein include, but are not limited to, diethylene glycol monoethyl ether (Transcutol®), Oleyl alcohol, Oleic acid, Azone (Laurocapram or 1-n-Dodecyl azacycloheptan-2-one), Propylene glycol mono- and diesters of fats and fatty acids (e.g. propylene glycol monocaprylate, propylene glycol monolaurate), Triglycerides and lipids (e.g. linoleic acid), Macrogolglycerides or Polyethylene glycol glycerides and fatty esters (e.g. stearoyl macrogolglycerides, oleoyl macrogolglycerides, lauroyl macrogolglycerides, Oleyl macrogol-6-glycerides, Lauroyl macrogol-6 glycerides), Glycerides and fatty acid esters of polyethylene glycol (e.g. caprylocaproyl macrogolglycerides, capryl-caproyl macrogolglycerides, oleoyl macrogol glycerides), Polyoxyl 40 Hydrogenated Castor Oil (Cremophor RH 40), Polysorbate 80 (Tween 80), Dodecylazacycloheptanone, SEPA^{®} such as described in US Patent 4,861,764 (e.g. 2-n-nonyl-1,3-dioxolane), and mixtures thereof. More preferred is diethylene glycol monoethyl ether (available from Gattefosse under the tradename Transcutol).

It is preferred that the formulations comprise from about 0.1% to about 99%, preferably from about 0.1% to about 90%, more preferably from about 5% to about 90%, even more preferably from about 15% to about 75%, by weight of skin penetration enhancer.

It is preferred that the ratio of photosensitizer to skin-penetration enhancer is from about 1:20 to about 1:10000, more preferably from about 1:60 to 1:300, on the basis of percentages by weight of total composition.

It is preferred that the photosensitizer is solubilised, especially when the photosensitizer is hydrophobic. One method of solubilising certain photosensitizers, including green porphyrins, is by formulation in liposomes or other lipid-containing complexes. An alternative may be to solubilise the photosensitizer in cyclodextrins or cyclodextrin derivatives. Preferred are partially etherified cyclodextrin, the ether substituents of which are hydroxyethyl, hydroxypropyl or dihydroxypropyl groups. However, appropriate cyclodextrins should be of a size and conformation appropriate for use with the photosensitizing agents disclosed herein..

Other methods suitable for solubilising certain photosensitizers include the use of a solvent acceptable for use in the treatment of skin tissues and cells such as, but are not limited to, DMSO (dimethylsulfoxide), polyethylene glycol (PEG) or any other solvent. It is preferred that the formulations herein comprise a solubilizer. Some solubilizers are also penetration enhancers and it is preferred that the formulations herein comprise a penetration enhancer that is also a solubilizer for the photosensitizer. Preferably the solubilizer is selected from glycol ethers, polyethylene glycol, polyethylene glycol derivatives, propylene glycol, propylene glycol derivatives, polysorbates (e.g. Tween™), fatty alcohols, aromatic alcohols, propylene glycol, glycerols, oils, surfactants, glucosides, and mixtures thereof. More preferably the solubilizer is selected from diethylene glycol monoethyl ether (Transcutol®), polyethylene glycol of average molecular weight from 100 to 5000, triethylene glycol, tetraethylene glycol, pentaethylene glycol, hexaethylene glycol, septaethylene glycol, octaethylene glycol, propylene glycol, propylene glycol mono- and diesters of fats and fatty acids (e.g. propylene glycol monocaprylate, propylene glycol monolaurate), benzyl alcohol, glycerol, oleyl alcohol, mineral oil, lanolin/lanolin derivatives, petrolatum or other petroleum products suitable for application to the skin, propylene glycol mono- and diesters of fats and fatty acids, macrogols, macrogolglycerides or polyethylene glycol glycerides and fatty esters (e.g. stearoyl macrogolglycerides, oleoyl macrogolglycerides, lauroyl macrogolglycerides, linoleoyl macrogolglycerides), ethoxylated castor oil (e.g. Cremophor - a polyoxyl hydrogenated castor oil), C6-C30 triglycerides, natural oils, glucosides (e.g. cetearyl glucoside), surfactants, and mixtures thereof. More preferable the solubilizer is selected from diethylene glycol monoethyl ether (Transcutol®), oleyl alcohol, and mixtures thereof.

It is preferred that the formulations herein comprise from about 0.1% to about 99%, more preferably from about 1% to about 75%, by weight of solubilizer.

It is preferred that the formulations have a viscosity at 20°C of from about 50 cps to about 50000 cps, more preferably from about 500 cps to about 40000 cps, even more preferably from about 5000 cps to about 30000 cps. Should the viscosity need to be adjusted it can be done by means of a viscosity modifying agent. Preferred viscosity modifiers are selected from polyethylene glycols, acrylic acid-based polymers (carbopol polymers or carbomers), polymers of acrylic acid crosslinked with allyl sucrose or allylpentaerythritol (carbopol homopolymers), polymers of acrylic acid modified by long chain (C10-C30) alkyl acrylates and crosslinked with allylpentaerythritol (carbopol copolymers), poloxamers also known as pluronics (block polymers; e.g. Poloxamer 124, 188, 237, 338, 407), waxes (paraffin, glyceryl monostearate, diethylene glycol monostearate, propylene glycol monostearate, ethylene glycol monosterate, glycol stearate), hard fats (e.g. Saturated C8-C18 fatty acid glycerides), xantham gum, polyvinyl alcohol, solid alcohols, and mixtures thereof.

Preferred formulations contain one or more PEGs. It is preferred that the formulation comprises at least one PEG of average molecular weight about 2000 or less, preferably about 1500 or less, preferably about 1000 or less, preferably about 800 or less, preferably about 600 or less, preferably about 500 or less, preferably about 400 or less. It is preferred that the formulation comprises at least one PEG of average molecular weight about 3000 or more, preferably about 3350 or more, preferably about 3500 or more. It is preferred that the formulation comprises a mixture of PEG's. More preferably, one PEG has an average molecular weight of about 800 or less and one PEG has an average molecular weight of 3000 or more.

A preferred formulation for use in the present invention comprises photosensitizer (especially green-porphyrins), low molecular weight PEG such as PEG200, diethylene glycol monoethyl ether (Transcutol®), high molecular weight PEG such as PEG3350 and fatty alcohol such as oleyl alcohol.

The formulation herein may comprise a variety of other components. Any suitable ingredient may be used herein but typically these optional component will render the formulations more cosmetically acceptable or provide additional usage benefits. Some examples of preferred optional ingredients include, but are not limited to, emulsifiers, humectants, emollients, surfactants, oils, waxes, fatty alcohols, dispersants, skin-benefit agents, pH adjusters, dyes/colourants, analgesics, perfumes, preservatives, and mixtures thereof.

Preparation of dry formulations that are reconstituted immediately before use also is contemplated. The preparation of dry or lyophilized formulations can be effected in a known manner, conveniently from the solutions of the invention. The dry formulations of this invention are also storable. By conventional techniques, a solution can be evaporated to dryness under mild conditions, especially after the addition of solvents for azeotropic removal of water, typically a mixture of toluene and ethanol. The residue is thereafter conveniently dried, *e.g*. for some hours in a drying oven.

For topical formulations (such as ointments) to be applied to the surface of the skin, the concentration of the photosensitizer in the excipient preferably ranges from about 0.001 to about 10% w/w, and more preferably from about 0.005 to about 5% w/w, and even more preferably between about 0.01 to about 1% w/w. Particularly preferred is the use of about a 0.2% w/w topical formulation.

Preferably sufficient time is left between delivery of the photosensitizer and administration of the activation energy to allow the photosensitizer to distribute within the target tissue. The exact length of time can vary according to the type of photosensitizer and the target tissue but, in general, it is preferred that 10 seconds or more, more preferably 1 minute or more, more preferably 5 minutes or more, is left between delivery of the photosensitizer and administration of the activation energy. Preferably the time between delivery of the drug and activation energy is 240 minutes or less, more preferably 180 minutes or less, even more preferably 60 minutes or less.

Preferably the photosensitizer is delivered in a topical composition and is left in contact with the skin for 5 to 60 minutes. Excess composition is then preferably removed by any suitable means. Preferred means include include wiping with dry cloth, wiping with a moist towelette, washing with alcohol, washing with a soap free cleanser, washing with a mild soap cleanser, and combinations thereof. Thereafter, it is preferred that the activation energy is delivered to the skin. This period will vary depending on the photosensitizer and the method of delivery. For example, lemuteporfin delivered topically can be activated shortly after application whereas ALA requires a delay while the ALA is metabolized into the photosensitive active.

Preferably, the activation energy comprises a wavelength close to at least one of the absorption peaks of the photosensitizer. This wavelength differs for different photosensitizers. For example, BPD-MA has an absorption peak at 689nm and so, when BPD-MA is the photosensitizer used, the wavelength of the activation energy is preferably is at or close to 689nm. The photosensitizer ALA-methyl ester (available under the tradename Metvix) has an absorption peak at 635nm and so when this photosensitizer is used the activation energy is preferable at or close to 635nm. ALA (available under the tradename Levulan) has an absorption peak at 417nm and at 630nm so when this photosensitizer is used the activation energy is preferable at or dose to 417nm and/or 630nm.

The activation energy herein may be provided by any suitable means. Generally, the activation energy is provided by a visible light source although it has been suggested that x-ray, ultraviolet, or ultrasound sources may be used. Preferred sources include, but are not limited to, lasers, light emitting diodes (LED), incandescent lamps, arc lamps, standard fluorescent lamps, U.V. lamps, and combinations thereof. More preferred are light emitting diodes. Alternatively, any convenient source of activation energy having a component of wavelengths that are absorbed by the photosensitizer may be used, for example, an operating room lamp, or any bright light source, including sunlight. Commercially available activation energy sources include CureLight™ (available from Photocure ASA, Oslo, Norway), BLU-U™ (available from DUSA, Wilmington, MA, USA), PDT Laser (available from Diomed, Andover, MA, USA), Ceralas™ (available from Biolitec AG, Jena, Germany), Omnilux PDT™ (available from PhotoTherapeutics Ltd., Birmingham, UK), and Q-Beam & Quanta-med (Quantum Devices Inc., Barneveld, WI, USA).

The activation energy dose administered during the PDT treatment contemplated herein can vary as necessary. Preferably, for photosensitizers of high potency, such as green porphyrins, the dosage of the light is about 25-100 J/cm². It is generally preferred that the total dose of the irradiation should generally not exceed 400 J/cm², preferably 200 J/cm², or more preferably not exceed 100 J/cm². Preferred doses can range between about 0.1 J/cm² to about 200 J/cm², more preferably 1 J/cm² to about 100 J/cm². For example, about 25, about 50, about 75, about 100, about 125, about 150, or about 175 J/cm². More preferred doses range from about 25 J/cm² to about 100J/cm².

Normally, the intensity of the energy source should not exceed about 600mW/cm². Irradiances between about 0.1 and 400 mW/cm² are preferred. Even more preferably the irradiance is between 5 and 100 mW/cm².

Normally, the irradiation lasts from about 10 seconds to about 4 hours, and preferably between about 5 minutes and 1 hour. For example, irradiation times of about 10, about 15, about 20, about 30, about 45, about 60, about 75, about 90, about 105, about 120, about 135, about 150, about 165 and about 180 minutes maybe used.

It is preferred that the area to be treated have minimal hair coverage when the activation energy is applied. Therefore, if there is significant hair coverage of the area to be treated, it is preferred that the hair is cut short or shaved prior to activation energy application. While not wishing to be bound by theory, it is believed that, due to the fact that hair has a shielding function, hair coverage can affect the activation energy dose that is delivered to the target area, especially when visible light wavelengths are used. Consequently, in order to more accurately deliver the correct does it is preferred that there be little or no hair coverage. Alternatively, the shielding effect of the hair may be compensated for by changes to delivery of the activation energy.

The irradiation or light exposure used in the invention may be directed to a small or large area of the body or face depending on the patch to be treated. Any part of the body may be treated but acne typically affects the face, chest, and/or back Treatment may be preceded with an assessment of the time of light exposure for the patient's minimal erythemal dose (MED) occurrence in order to avoid potential burning of the exposed skin.

The treatment maybe repeated as many times as is necessary. If repeated, the treatment frequency may vary. For example, the treatments could be daily, everytwo days, twice weekly, weekly, every two weeks, twice monthly, every four weeks, monthly, every six weeks, every eight weeks, every two months, quarterly, twice annually, or annually, or other suitable time interval. Preferably the treatment is not repeated more than once per week, even more preferably not more than once every two weeks. Preferably, the treatment is repeated at least once every six months. More preferably at least once every three months. Even more preferably at least once every two months. The total number of treatments can range from one to as many as required. It is preferred that the total number of treatments in any 6 month period be from 1 to 12, more preferablyfrom 1 to 6, even more preferably from 2 to 3.

A preferred regimen according to the present invention comprises:
a) administering photosensitizer topically to the acne affected skin. Preferably the composition comprises photosensitizer and skin-penetration enhancer. The preferred photosensitizer is selected from verteporfin, lemuteporfin, and combinations thereof.
b) administering activation energy via LED's. Preferably, the activation energy is administered within 60 minutes of application. Preferred doses are between 15 and 200 J/cm². More preferred doses include 20, 40, 80, or 120 J/cm².

### EXAMPLES

It will be understood that the following embodiments of the present invention are intended to be illustrative of some of the possible applications or principles. Various modifications may be made by the skilled person without departing from the true spirit and scope of the invention.

Patients with moderate to severe acne as defined by the presence of pustular and or cystic lesions, with or without scarring, are assessed for PDT treatment with lemuteporfin 0.2% ointment. Prior to treatment, the areas to be treated with PDT are cleansed and cleared of any hair, skin lotions or cosmetic products.

Topical photosensitizer ointment (comprising 0.2 wt% lemuteporfin, 50 wt% PEG-200, 24 wt% Transcutol^{®} , 10wt% PEG-3350 and 15.8 wt% oleyl alcohol) is applied directly on the acne affected skin at a quantity of approximately 45 mg / cm2. The ointment is left on for absorption for 20-45 minutes. Immediately prior to light treatment, excess ointment is removed by gentle wiping with a water-based skin cleanser. Acne lesions and the immediate surrounding areas are illuminated with 689 nm PDT light at a dose of 100J/cm² with an intensity of 50mW/cm².

The present invention provides:
1. A method of treating acne vulgaris by:
   (i) topically applying a composition comprising a green porphyrin photosensitizer and solublizer to skin tissue exhibiting symptoms of acne, and
   (ii) exposing the tissue to energy at a wavelength capable of activating the photosensitizer.
2. A method according to embodiment 1 wherein the photosensitizer is selected from verteporfin, lemuteporfin, and combinations thereof.
3. A method according to embodiment 1 or 2 wherein the composition has a viscosity at 20°C of from about 50 cps to about 50000 cps.
4. A method of treating acne vulgaris by:
   (i) topically applying a composition comprising a photosensitizer to skin tissue exhibiting symptoms of acne,
   (ii) removing excess composition from the skin, and
   (iii) exposing the tissue to energy at a wavelength capable of activating the photosensitizer.
5. A method according to embodiment 4 where the photosensitizer is selected from green porphyrins.
6. A method according to embodiment 4 wherein the photosensitizer is selected from verteporfin, lemuteporfin, and combinations thereof.
7. A method according to embodiment 4, 5, or 6 wherein the excess composition is removed by wiping with dry cloth, wiping with a moist towelette, washing with alcohol, washing with a soap free cleanser, washing with a mild shampoo, and combinations thereof.
8. A method of treating acne vulgaris by:
   (i) topically applying a composition comprising a green porphyrin and solublizer to skin tissue exhibiting symptoms of acne, and
   (ii) exposing the tissue to energy at a wavelength capable of activating the photosensitizer,
      wherein the is repeated until the total number of acne lesions has been reduced by 30% or more.
9. A method of treating acne vulgaris by:
   (i) photodynamically treating skin exhibiting symptoms of acne, and
   (ii) treating the same patient with topical retinoids, oral retinoids, systemic antibiotics, topical or local antibiotics, oral contraceptives, topical anti- androgens, blue light therapy, laser therapy, or combinations thereof.
10. A method according to embodiment 9 wherein the photodynamic therapy is carried out according to any of embodiments 1 to 7.
11. A method according to embodiment 9 or 10 wherein the non-photodynamic therapy is selected from topical retinoids, oral retinoids, topical antibiotics, topical anti-androgens, or combinations thereof.
12. A method according to embodiment 9 or 10 wherein the non-photodynamic therapy is selected from topical retinoids.
13. A method of treating acne vulgaris by:
   (i) topically applying a composition comprising a green porphyrin and skin-penetration enhancer to skin tissue exhibiting symptoms of acne, and
   (ii) exposing the tissue light supplied by a light emitting diode device wherein the device comprises LED's emitting red light and LED's emitting blue light.
14. A method according to embodiment 13 wherein the red-light emitting LED's emit in the range 600-750nm and blue-light emitting LED's emit in the range 390-450nm.
15. The use of a green porphyrin photosensitizer and solublizer to prepare a topical medicament for treating acne vulgaris, wherein the photosensitizer is capable of being activated by energy of a selected wavelength.
16. The use of a photosensitizer according to embodiment 15, wherein the photosensitizer is selected from verteporfin, lemuteporfin, and combinations thereof.
17. The use of a photosensitizer according to embodiment 15 or 16, wherein the medicament has a viscosity at 20°C of from about 50 cps to about 50000 cps.
18. The use of a photosensitizer according to embodiment 15, wherein the photosensitizer is selected from green porphyrins.
19. The use of a photosensitizer according to any one of embodiments 15 to 18, wherein the wavelength is in the range 600 to 750 nm or 390-450nm.

## Claims

1. A green porphyrin photosensitizer and solublizer for use in a method of treating acne vulgaris by:
(i) topically applying a composition comprising a green porphyrin photosensitizer and solublizer to skin tissue exhibiting symptoms of acne, and
(ii) exposing the tissue to energy at a wavelength capable of activating the photosensitizer.

2. A green porphyrin photosensitizer and solublizer for use according to Claim 1, wherein the composition has a viscosity at 20°C of from about 50 cps to about 50000 cps.

3. A photosensitizer for use in a method of treating acne vulgaris by:
(i) topically applying a composition comprising a photosensitizer to skin tissue exhibiting symptoms of acne,
(ii) removing excess composition from the skin, and
(iii) exposing the tissue to energy at a wavelength capable of activating the photosensitizer.

4. A photosensitizer for use according to Claim 3, wherein the photosensitizer is selected from green porphyrins.

5. A green porphyrin photosensitizer and solublizer for use according to Claim 1 or a photosensitizer for use according to Claim 3, wherein the photosensitizer is selected from verteporfin, lemuteporfin, and combinations thereof.

6. A photosensitizer for use according to Claim 3, Claim 4 or Claim 5 when dependent on Claim 4, wherein the excess composition is removed by wiping with dry cloth, wiping with a moist towelette, washing with alcohol, washing with a soap free cleanser, washing with a mild shampoo, and combinations thereof.

7. A green porphyrin and a solublizer for use in a method of treating acne vulgaris by:
(i) topically applying a composition comprising a green porphyrin and solublizer to skin tissue exhibiting symptoms of acne, and
(ii) exposing the tissue to energy at a wavelength capable of activating the photosensitizer,
wherein the administration is repeated until the total number of acne lesions has been reduced by 30% or more.

8. A green porphyrin photosensitizer and solublizer for use according to any one of Claims 1, 2 or 5 or a photosensitizer for use according to any one of Claims 3 to 6, wherein the method further comprises treating the same patient with topical retinoids, oral retinoids, systemic antibiotics, topical or local antibiotics, oral contraceptives, topical anti-androgens, blue light therapy, laser therapy, or combinations thereof.

9. A green porphyrin photosensitizer and solublizer or a photosensitizer for use according to Claim 8, wherein the non-photodynamic therapy is selected from topical retinoids.

10. A green porphyrin and skin penetration enhancer for use in a method of treating acne vulgaris by:
(i) topically applying a composition comprising a green porphyrin and skin penetration enhancer to skin tissue exhibiting symptoms of acne, and
(ii) exposing the tissue light supplied by a light emitting diode device wherein the device comprises LED's emitting red light and LED's emitting blue light.

11. A green porphyrin and skin penetration enhancer for use according to Claim 10 wherein the red-light emitting LED's emit in the range 600-750nm and blue-light emitting LED's emit in the range 390-450nm.

12. Use of a green porphyrin photosensitizer and solublizer in the manufacture of a medicament for the treatment of acne vulgaris.

13. Use of a photosensitizer in the manufacture of a medicament for the treatment of acne vulgaris, preferably wherein the photosensitizer is selected from green porphyrins.

14. Use of a green porphyrin and skin-penetration enhancer in the manufacture of a medicament for the treatment of acne vulgaris.

15. A use according to any one of claims 12 to 14, wherein:
(a) the photosensitizer is selected from verteporfin, lemuteporfin, and combinations thereof; and/or
(b) the medicament has a viscosity at 20°C of from about 50 cps to about 50000 cps; and/or
(c) the photosensitizer is capable of being activated by energy of a selected wavelength, preferably wherein the wavelength is in the range 600 to 750 nm or 390-450 nm.
